# EUROPÄISCHE PATENTSCHRIFT

(11) **EP 1 419 793 B1**
(45) Veröffentlichungstag und Bekanntmachung des Hinweises auf die Patenterteilung: **25.01.2006**
(21) Anmeldenummer: 03090354.6
(22) Anmeldetag: 17.10.2003
(51) Int. Cl.: A61L 31/02

(54) **Endoprothese mit einer Trägerstruktur aus einer Magnesiumlegierung**
Endoprosthesis with a supporting structure of magnesium alloy
Endoprothese avec une structure porteuse en alliage de magnesium

(30) Priorität: 13.11.2002 DE 10253634
(43) Veröffentlichungstag der Anmeldung: 19.05.2004
(73) Patentinhaber: Biotronik AG, 8180 Bülach (CH)
(72) Erfinder: Harder, Claus, Dr., 91080 Uttenreuth (DE); Gerold, Bodo, Dr., 97267 Himmelstadt (DE); Müller, Heinz, Dr., 91054 Erlangen (DE); Heublein, Bernd, Prof. Dr., 30627 Hannover (DE)
(74) Vertreter: Eisenführ, Speiser & Partner

(56) Entgegenhaltungen:
- FR-A- 1 412 298
- US-A- 3 687 135
- US-B1- 6 287 332

## Beschreibung

Die Erfindung betrifft eine Endoprothese mit einer Tragstruktur, welche einen metallischen Werkstoff enthält. Die Erfindung betrifft insbesondere intraluminale Endoprothesen wie Stents.

Viele Endoprothesen haben den Zweck, im Inneren des Körpers eines Patienten eine Stützfunktion zu übernehmen. Dementsprechend sind Endoprothesen implantierbar ausgeführt und besitzen eine Tragstruktur, die die Stützfunktion gewährleistet. Bekannt sind Implantate aus metallischen Werkstoffen. Die Wahl von Metallen als Werkstoff für die Tragstruktur eines derartigen Implantats beruht vor allem auf den mechanischen Eigenschaften von Metallen.

In einigen Fällen, insbesondere im Falle solcher intraluminaler Endoprothesen wie Stents ist eine dauerhafte Stützfunktion durch die Endoprothese nicht erforderlich. Vielmehr kann sich in einigen dieser Anwendungsfälle das Körpergewebe unter Anwesenheit der Stützprothese derart erholen, dass eine dauerhafte Stützwirkung durch die Prothese nicht erforderlich ist. Dies hat zu dem Gedanken geführt, solche Prothesen aus bioresorbierbarem Material zu fertigen.

Insbesondere in der DE 197 31 021 ist ein bioresorbierbarer Metallstent bekannt, dessen Werkstoff als Hauptbestandteil Magnesium, Eisen oder Zink enthält.

Insbesondere metallische Stents sind in großer Zahl bekannt. Einer der Hauptanwendungsbereiche solcher Stents ist das dauerhafte Weiten und Offenhalten von Gefäßverengungen, insbesondere von Verengungen (Stenosen) der Herzkranzgefäße. Daneben sind auch Aneurysmenstents bekannt, die eine Stützfunktion für eine beschädigte Gefäßwand bieten. Derartige Stents besitzen in der Regel eine Umfangswandung von ausreichender Tragkraft, um das verengte Gefäß im gewünschten Maße offen zu halten. Um einen ungehinderten Blutfluss durch den Stent zu ermöglichen, ist diese an beiden Stirnseiten offen. Die tragende Umfangswandung wird in der Regel von einer gitterartigen Tragstruktur gebildet, die es erlaubt, den Stent in einem komprimierten Zustand mit kleinem Außendurchmesser bis zur zu behandelnden Engstelle des jeweiligen Gefäßes einzuführen und dort beispielsweise mit Hilfe eines Ballonkatheters soweit aufzuweiten, dass das Gefäß unter Anwesenheit des Stents nach entfernen des Ballonkatheters den gewünschten, vergrößerten Innendurchmesser aufweist. An den Stent wird daher grundsätzlich die Anforderung gestellt, dass seine Tragstruktur im aufgeweiteten Zustand eine ausreichende Tragkraft aufweist, um das Gefäß offen zu halten. Um unnötige Gefäßbeschädigungen zu vermeiden, ist außerdem gewünscht, dass der Stent nach dem Aufweiten nach Entfernen des Ballons nur wenig elastisch zurückfedert (Recoil), um den Stent beim Aufweiten nur möglichst wenig über den gewünschten Enddurchmesser hinaus weiten zu müssen. Weitere Kriterien, die in Bezug auf einen Stent wünschenswert sind, sind beispielsweise eine gleichmäßige Flächenabdeckung, eine Struktur, die eine gewisse Flexibilität in Bezug auf die Längsachse des Stents erlaubt und dergleichen.

Neben den gewünschten mechanischen Eigenschaften eines Stents soll dieser möglichst in einer Weise mit dem Körpergewebe am Implantationsort interagieren, dass es nicht zu erneuten Gefäßverengungen, insbesondere durch den Stent selbst hervorgerufenen Gefäßverengungen kommt. Eine Restenose (Wiederverengung des Gefäßes) soll möglichst vermieden werden. Auch ist es wünschenswert, wenn von dem Stent möglichst gar keine oder nur eine sehr geringe inflammatorische Wirkung ausgeht. In Bezug auf einen biodegradierbaren Metallstent ist es darüber hinaus wünschenswert, dass von den Abbauprodukten des Metallstents möglichst wenig negative physiologische Wirkungen und wenn möglich sogar positive physiologische Wirkungen ausgehen.

Vor diesem Hintergrund liegt der vorliegenden Patentanmeldung die Aufgabe zugrunde, eine Endoprothese der Eingangs genannten Art hinsichtlich ihrer Eigenschaften zu optimieren.

Erfindungsgemäß wird diese Aufgabe durch eine Endoprothese der Eingangs genannten Art gelöst, deren metallischer Werkstoff eine Magnesiumlegierung folgender Zusammensetzung enthält:

| | |
|---|---|
| Magnesium: | > 90 % |
| Yttrium: | 3,7 % - 5,5 % |
| Seltene Erden: | 1,5 % - 4,4 % |
| Rest: | < 1 % |

Diese Lösung beruht auf der unerwartenden Erkenntnis, dass eine Endoprothese, die ganz oder teilweise aus der genannten Magnesiumlegierung besteht, hinsichtlich der vielfältigen, wünschenswerten Eigenschaften viele der Anforderungen in ganz besonders positiver Weise erfüllt. Neben den mechanischen Anforderungen erfüllt ein Werkstoff oft ganz oder teilweise aus der genannten Magnesiumlegierung auch die weiteren physiologischen Eigenschaften, als da sind geringe inflammatorische Wirkung und nachhaltige Verhinderung von Gewebewucherungen wie beispielsweise Restenosen. In der Tat haben Versuche ergeben, dass die Abbauprodukte der genannten Magnesiumlegierung nicht nur wenig oder gar keine negativen physiologischen Wirkungen haben, sondern prima facie sogar positive Eigenschaften. Daher stellt die genannte Magnesiumlegierung unter der Vielzahl der denkbaren Werkstoffe eine unerwartet glückliche Auswahl dar.

Vorzugsweise beträgt der Yttriumanteil der Magnesiumlegierung zwischen 4 % und 5 %. Der Anteil Seltener Erden an der Magnesiumlegierung liegt vorzugsweise zwischen 1,5 % und 4 % ein bevorzugtes Seltene-Erden-Element ist Neodym. Der Restanteil an der Magnesiumlegierung von unter 1 % ist vorzugsweise zum größten Teil von Zirkonium und daneben möglicherweise von Lithium gebildet.

Aufgrund der außerordentlich positiven Eigenschaften der genannten Magnesiumlegierung besteht die Tragstruktur der Endoprothese vorzugsweise zur Gänze aus der Magnesiumlegierung.

Der Werkstoff der Tragstruktur ist vorzugsweise stranggepresst. Es hat sich herausgestellt, dass die Verarbeitung des Werkstoffes dessen physiologische Wirkung beeinflusst. In diesem Sinne wird eine Tragstruktur bevorzugt, welche die folgenden physiologischen Eigenschaften in einschlägig bekannten Zelltests aufweist: in dem Vitalitätstest MTS über 70% Absorption bei 490 nm bezogen auf glatte Muskelzellen (koronare Endothel-Zellen) mit 100%, d.h. eine Zellüberlebensrate von über 70% bei Kultivierung der Zellen mit einem Eluat des Werkstoffs der Tragstruktur im Vergleich zu unbehandelten Zellen. In dem Proliferationstest mit BrdU (Bromdeoxyuridin) ergibt sich eine Proliferationshemmung auf unter 20% bezogen auf unbehandelte glatte Muskelzellen, d.h. unter dem Einfluss der Magnesiumlegierung der Tragstruktur beträgt die Zahl der aufgrund der Aufnahme von BrdU fluorezierenden Zellen 20% bezogen auf eine Gesamtheit von 100% in dem Vergleichstest mit unbehandelten Muskelzellen. Während beispielsweise stranggepresste Tragstrukturen aus der Magnesiumlegierung diese physiologischen Eigenschaften besitzen, hat sich herausgestellt eine gegossene Tragstruktur diese Eigenschaften nicht besitzt. Diese physiologischen Eigenschaften sind somit zumindest zum Teil durch den Herstellungsprozess bedingt und nicht notwendigerweise inherente Eigenschaften der Magnesiumlegierung. Ein Einflussfaktor ist auch die Wärmebehandlung der Magnesiumlegierung während der Verarbeitung zur fertigen Tragstruktur.

Die Endoprothese ist vorzugsweise als intraluminale Endoprothese ausgebildet. Besonders bevorzugt ist eine Endoprothese, die als Stent, und zwar insbesondere als Koronarstent oder als peripherer Stent ausgebildet ist. Koronarstents, die die genannte Magnesiumlegierung enthalten, haben im Versuch eine Summe unerwartet positiver Eigenschaften gezeigt.

Vor allen für eine Tragstruktur für Stents bietet es sich an, diese in an sich bekannter Weise entweder als selbstexpandierende Tragstruktur oder als ballonexpandierbare Tragstruktur zu gestalten. Für eine ballonexpandierbare Tragstruktur kommt vor allem eine Herstellung aus einem Rohr in Frage, welches beispielsweise mit Hilfe eine Lasers geschnitten wird. Für eine selbstexpandierende Tragstruktur aus Magnesiumlegierung bietet sich ein Drahtstent an, der aus Draht geformt ist, welcher die Magnesiumlegierung enthält.

Die Tragstruktur ist vorzugsweise gitterartig ausgebildet und wird von Stegen sowie von von den Stegen umschlossenen, radialen Öffnungen gebildet. Diese Stege haben vorzugsweise derart ähnliche Querschnittsflächen, dass das Verhältnis von größter zu kleinster Querschnittsfläche kleiner als 2 ist. Ähnliche Steg-Querschnittsflächen führen dazu, dass das Implantat in allen Bereichen etwa gleichmäßig schnell abgebaut wird.

Dem gleichmäßigen Abbau des Implantats dienen auch Stege, bei denen das Verhältnis vom größten zum kleinsten Minimalquerschnitt - im Sinne eines jeweils kleinsten Durchmessers - kleiner als 3 ist.

Im Falle eines bevorzugten Aufbaus eines Stents aus Stegringen, die durch Verbindungsstege verbunden sind, haben die Verbindungsstege vorzugsweise eine kleinere Querschnittsfläche oder einen kleineren minimalen Durchmesser, als die Stege, die die Stegringe bilden. Dadurch wir erreicht, dass die Verbindungsstege im Körper eines Patienten schneller abgebaut werden, als die Stegringe. Dies wiederum hat zur Folge, dass eine axiale Flexibilität des Stents durch Abbau der Verbindungsstege schneller zunimmt, als die Tragkraft des Stents in Folge des Abbaus der Stegringe abnimmt. Dieses Merkmal, Verbindungsstege im Vergleich zu tragenden Stegen dünner zu gestalten, ist nicht nur im Zusammenhang mit den hier interessierenden Magnesium-Stents sondern im Zusammenhang mit jeder Art von bioresorbierbaren Stents von selbständiger, erfinderischer Bedeutung.

Schließlich sind Endoprothesen bevorzugt, die einen physiologisch wirksamen Wirkstoff tragen, insbesondere wenigstens mit einem Medikament beschichtet sind.

Die Erfindung soll nun anhand eines Ausführungsbeispiels mit Hilfe der Figuren näher erläutert werden. Von den Figuren zeigen:
Figur 1 eine schematische Darstellung einer Endoprothese in Form eines Stents;
Figur 2 eine Abwicklung der Tragstruktur des Stents aus Figur 1 und
Figur 3 einen Querschnitt durch einen Steg der Tragstruktur aus Figur 2.

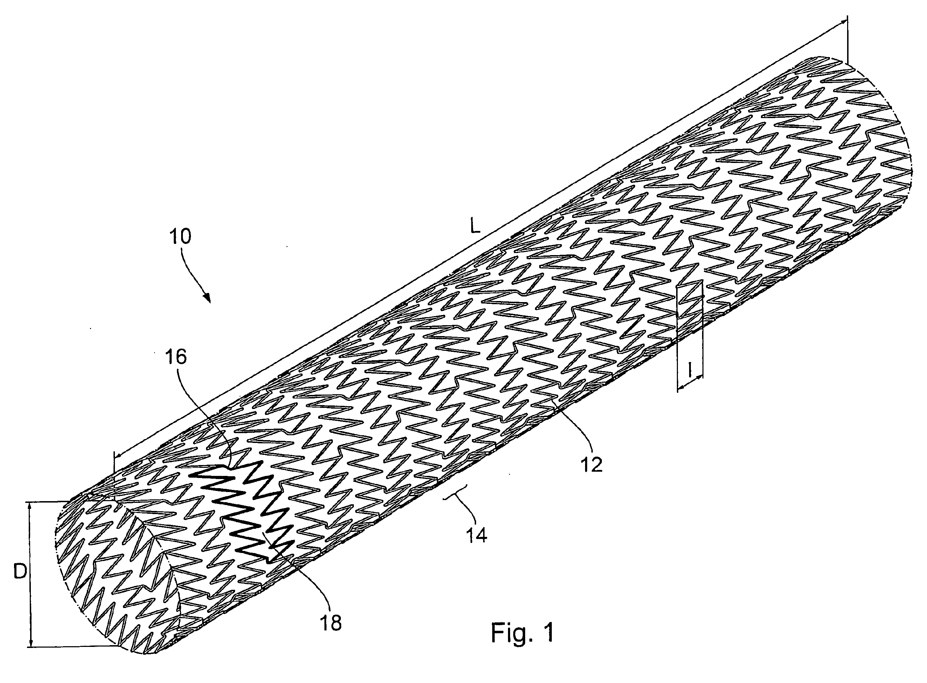

Figur 1 zeigt eine Endoprothese als endoluminale Prothese in Form eines Stents mit einer Tragstruktur 10. Dieser Stent und seine Tragstruktur 10 haben die Form eines an seinen Stirnseiten offenen Hohlköpers, dessen Umfangswandung von der Tragstruktur 10 gebildet ist, die wiederum von teilweise gefalteten Stegen 12 gebildet ist. Die Stege 12 bilden Stützabschnitte 14, welche von jeweils einem in Längsrichtung ringförmig geschlossenen, zick-zack- oder mäanderförmig gefalteten Steg 12 gebildet sind.

Die Tragstruktur 10 des Stents wird von mehreren solcher in Längsrichtung aufeinanderfolgender Stützabschnitte 12 gebildet. Die Stützabschnitte bzw. Stegringe 14 sind über Verbindungsstege 16 miteinander verbunden. Jeweils zwei in Umfangsrichtung aneinander benachbarte Verbindungsstege 16 sowie die zwischen diesen Verbindungsstegen 16 einander gegenüberliegenden Teilabschnitte der Stegringe oder Stützabschnitte 14 definieren eine Masche 18 des Stents 10. Eine solche Masche 18 ist in Figur 1 hervorgehoben dargestellt. Jede Masche 18 umschließt eine radiale Öffnung der Umfangswandung bzw. der Tragstruktur des Stents 10.

Jeder Stegring 14 weist etwas drei bis sechs über den Umfang des Stents 10 gleich verteilte Verbindungsstege 16 auf, die jeweils einen Stegring 14 mit dem benachbarten Stegring 14 verbinden. Dementsprechend weist der Stent 10 in Umfangsrichtung zwischen zwei Stützabschnitten 14 jeweils drei bis sechs Maschen auf.

Aufgrund der Faltung der Stege 12 ist der Stent 10 in Umfangsrichtung expandierbar. Dies geschieht beispielsweise mit einem an sich bekannten Ballonkatheter, der an seinem distalen Ende einen mittels eines Fluids expandierbaren Ballon aufweist. Der Stent 10 ist im komprimierten Zustand auf den deflatierten Ballon aufgecrimpt. Mit Expansion des Ballons werden sowohl der Ballon als auch der Stent 10 aufgeweitet. Anschließend kann der Ballon wieder deflatiert werden und der Stent 10 löst sich von dem Ballon. Auf diese Weise kann der Katheter gleichzeitig dem Einführen des Stents 10 in ein Blutgefäß und insbesondere in ein verengtes Herzkranzgefäß sowie zum Expandieren des Stents an diesem Ort dienen.

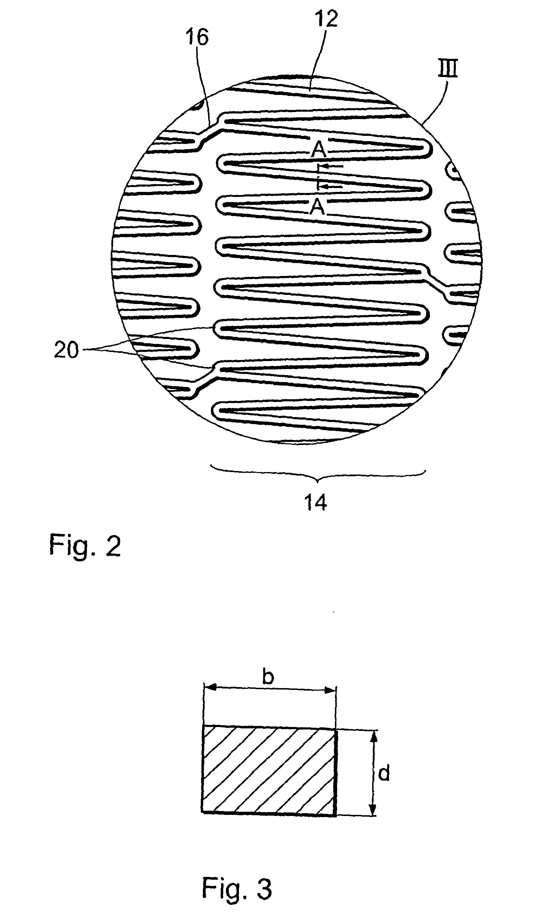

In Figur 2 ist ein Ausschnitt aus einer Abwicklung der Umfangswandung des Stents 10 dargestellt. Die Abwicklung zeigt den komprimierten Zustand des Stents 10.

In Figur 3 ist der in Figur 2 eingezeichnete Schnitt A-A durch einen Steg 12 des Stents 10 dargestellt. Es ist zu erkennen, dass der Steg 12 einen rechteckigen Querschnitt besitzt und in radialer Richtung bezogen auf den Stent eine Dicke d aufweist. Die Ausdehnung eines Steges 12 in Umfangsrichtung des Stents ist die Breite b.

In bevorzugten Ausführungsvarianten des Stents haben die Stege 12 alle eine in etwa ähnliche Querschnittsfläche, so dass zu mindest das Verhältnis von größter zu kleinster Querschnittsfläche nicht größer ist als zwei.

Auch sollte jeweils die kleinste Ausdehnung der Stege 12 des Stents 10 - je nach dem b oder d - für den gesamten Stent entweder in dem Sinne gleich sein, dass das Verhältnis der relativ größten kleinsten Ausdehnung eines Steges 12 an einem Ort des Stents 10 zur relativ kleinsten kleinsten Ausdehnung eines Stegs 12 an einem anderen Ort des Stents 10 kleiner ist als zwei.

Die Verbindungsstege 16 haben einen kleineren Querschnitt als die Stege 12. Sie sind insbesondere dünner, das heißt, das Maß d ist geringer als bei den Stegen 12. Dies hat zur Folge, dass die Verbindungsstege die ersten sind, die im Körper eines Patienten abgebaut werden. Damit erhöht sich die axiale Biegsamkeit des Stents bei gleichzeitig weiterhin durch die Stegringe 14 gegebener Stützwirkung des Stents. Die Stützwirkung des Stents 10 nimmt durch den langsameren Abbau der Stegringe 14 im Vergleich zu den Verbindungsstegen 16 langsamer ab, als die axiale Flexibilität zunimmt.

Die Tragstruktur des in den Figuren dargestellten Stents 10 besteht aus einer Magnesiumlegierung, deren Magnesiumanteil größer ist als 90 %. Daneben umfasst die Magnesiumlegierung Yttrium mit einem Anteil von 4 % bis 5 % sowie Neodym als Seltene-Erden-Element mit einem Anteil von 1,5 % bis 4%. Die Restbestandteile der Legierung sind kleiner als 1 % und werden zum größten Teil von Lithium oder Zirkonium gebildet.

## Patentansprüche

1. Endoprothese mit einer Tragstruktur (10), welche einen metallischen Werkstoff enthält, **dadurch gekennzeichnet, dass** der metallische Werkstoff eine Magnesiumlegierung folgender Zusammensetzung enthält:
| | |
|---|---|
| Magnesium: | > 90% |
| Yttrium: | 3,7% - 5,5% |
| Seltene Erden: | 1,5% - 4,4% |
| Rest: | < 1% |

2. Endoprothese nach Anspruch 1, **dadurch gekennzeichnet, dass** der YttriumAnteil der Magnesiumlegierung zwischen 4% und 5% beträgt.

3. Endoprothese nach Anspruch 1, **dadurch gekennzeichnet, dass** der Anteil seltener Erden an der Magnesiumlegierung zwischen 1,5% und 4% beträgt.

4. Endoprothese nach Anspruch 1, **dadurch gekennzeichnet, dass** der Anteil seltener Erden an der Magnesiumlegierung von Neodym gebildet ist.

5. Endoprothese nach Anspruch 1, **dadurch gekennzeichnet, dass** der RestAnteil an der Magnesiumlegierung zum größten Teil von Zirkonium gebildet ist.

6. Endoprothese nach einem der Ansprüche 1 bis 5, **dadurch gekennzeichnet, dass** die Tragstruktur (10) aus einer Magnesium-Legierung gemäß der Ansprüche 1 bis 5 besteht.

7. Endoprothese nach einem der Ansprüche 1 bis 6, **dadurch gekennzeichnet, dass** die Tragstruktur stranggepresst ist

8. Endoprothese nach Anspruch 1, **dadurch gekennzeichnet, dass** die Endoprothese als intraluminale Endoprothese ausgebildet ist.

9. Endoprothese nach Anspruch 8, **dadurch gekennzeichnet, dass** die Endoprothese als Stent ausgebildet ist.

10. Endoprothese nach Anspruch 9, **dadurch gekennzeichnet, dass** die Endoprothese als Koronarstent oder als peripherer Stent ausgebildet ist.

11. Endoprothese nach Anspruch 9 oder 10, **dadurch gekennzeichnet, dass** die Endoprothese als selbstexpandierender oder als ballonexpandierbarer Stent ausgebildet ist.

12. Endoprothese nach Anspruch 1, **dadurch gekennzeichnet, dass** die Tragstruktur Schneiden eines Rohres aus einem Stück hergestellt ist.

13. Endoprothese nach Anspruch 1, **dadurch gekennzeichnet, dass** die Tragstruktur aus einem Draht geformt ist, der die Magnesiumlegierung enthält.

14. Endoprothese nach Anspruch 1, **dadurch gekennzeichnet, dass** die Tragstruktur (10) einen längsgestreckten Hohlraum umschließt, welcher an seinen Stirnseiten offen ist.

15. Endoprothese nach Anspruch 14, **dadurch gekennzeichnet, dass** die Tragstruktur (10) gitterartig ausgebildet und von Stegen (12, 16) sowie von Stegen umschlossenen, radialen Öffnungen gebildet ist.

16. Endoprothese nach Anspruch 15, **dadurch gekennzeichnet, dass** die Stege (12) der Tragstruktur sämtlich eine derart ähnliche Querschnittsfläche aufweisen, dass das Verhältnis von größter zu kleinster Querschnittsfläche kleiner als 2 ist.

17. Endoprothese nach Anspruch 15, **dadurch gekennzeichnet, dass** die Stege (12) der Tragstruktur sämtlich eine derart ähnlichen minimalen Durchmesser aufweisen, dass das Verhältnis von größtem zu kleinstem minimalen Durchmesser kleiner als 2 ist.

18. Endoprothese nach Anspruch einem der Ansprüche 15 bis 17, mit einer Tragstruktur (10), die von Stegen (12) gebildete Stegringe (14) und benachbarte Stegringe miteinander verbindende Verbindungsstege (16) umfasst, **dadurch gekennzeichnet, dass** die Verbindungsstege (16) eine kleinere Querschnittsfläche oder einen kleineren minimalen Durchmesser haben, als die Stege (12), welche die Stegringe (14) bilden.

19. Endoprothese nach Anspruch 1', **dadurch gekennzeichnet, dass** die Endoprothese einen physiologisch wirksamen Wirkstoff trägt.

20. Endoprothese nach Anspruch 19, **dadurch gekennzeichnet, dass** die Endoprothese mit wenigstens einem Medikament beschichtet ist.

## Claims

1. Endoprosthesis having a carrier structure (10) comprising a metallic material, **characterised in that** the metallic material comprises a magnesium alloy of the following composition:
| | |
|---|---|
| Magnesium: | > 90 % |
| Yttrium: | 3.7 % to 5.5 % |
| Rare earths: | 1.5 % to 4.4 % |
| Remainder: | < 1 % |

2. Endoprosthesis according to claim 1, **characterised in that** the yttrium content of the magnesium alloy is between 4 % and 5 %.

3. Endoprosthesis according to claim 1, **characterised in that** the rare earths content of the magnesium alloy is between 1.5 % and 4 %.

4. Endoprosthesis according to claim 1, **characterised in that** the rare earths content of the magnesium alloy is formed by neodymium.

5. Endoprosthesis according to claim 1, **characterised in that** the remaining content of the magnesium alloy is formed for the most part by zirconium.

6. Endoprosthesis according to any one of claims 1 to 5, **characterised in that** the carrier structure (10) consists of a magnesium alloy according to claims 1 to 5.

7. Endoprosthesis according to any one of claims 1 to 6, **characterised in that** the carrier structure is extruded.

8. Endoprosthesis according to claim 1, **characterised in that** the endoprosthesis is in the form of an intraluminal endoprosthesis.

9. Endoprosthesis according to claim 8, **characterised in that** the endoprosthesis is in the form of a stent.

10. Endoprosthesis according to claim 9, **characterised in that** the endoprosthesis is in the form of a coronary stent or a peripheral stent.

11. Endoprosthesis according to either claim 9 or claim 10, **characterised in that** the endoprosthesis is in the form of a self-expanding stent or a balloon-expandable stent.

12. Endoprosthesis according to claim 1, **characterised in that** the carrier structure is produced by cutting a tube from one piece.

13. Endoprosthesis according to claim 1, **characterised in that** the carrier structure is formed by a wire which contains the magnesium alloy.

14. Endoprosthesis according to claim 1, **characterised in that** the carrier structure (10) encloses an elongated cavity which is open at its end faces.

15. Endoprosthesis according to claim 14, **characterised in that** the carrier structure (10) is of a lattice-like structure and is formed by webs (12, 16) and radial openings enclosed by webs.

16. Endoprosthesis according to claim 15, **characterised in that** the webs (12) of the carrier structure all have a similar cross-sectional area such that the ratio of the largest to the smallest cross-sectional area is less than 2.

17. Endoprosthesis according to claim 15, **characterised in that** the webs (12) of the carrier structure all have a similar minimum diameter such that the ratio of the largest to the smallest minimum diameter is less than 2.

18. Endoprosthesis according to any one of claims 15 to 17, having a carrier structure (10), which comprises web rings (14) formed by webs (12) and connecting webs (16) for interconnecting adjacent web rings, **characterised in that** the connecting webs (1,6) have a smaller cross-sectional area or a smaller minimum diameter than the webs (12) which form the web rings (14).

19. Endoprosthesis according to claim 1, **characterised in that** the endoprosthesis contains a physiologically effective active substance.

20. Endoprosthesis according to claim 19, **characterised in that** the endoprosthesis is coated with at least one pharmaceutical composition.

## Revendications

1. Endoprothèse avec une structure porteuse (10) contenant un matériau métallique, **caractérisée en ce que** le matériau métallique contient un alliage de magnésium de composition suivante :
| | |
|---|---|
| Magnésium : | > 90 % |
| Yttrium : | 3,7 %- 5,5 % |
| Terres rares : | 1,5 % - 4,4 % |
| Reste : | < 1 % |

2. Endoprothèse selon la revendication 1, **caractérisée en ce que** la part d'yttrium de l'alliage de magnésium est comprise entre 4 % et 5 %.

3. Endoprothèse selon la revendication 1, **caractérisée en ce que** la part de terres rares de l'alliage de magnésium est comprise entre 1,5 % et 4 %.

4. Endoprothèse selon la revendication 1, **caractérisée en ce que** la part de terres rares de l'alliage de magnésium est constituée de néodyme.

5. Endoprothèse selon la revendication 1, **caractérisée en ce que** la part restante de l'alliage de magnésium est principalement constituée de zirconium.

6. Endoprothèse selon l'une des revendications 1 à 5, **caractérisée en ce que** la structure porteuse (10) est constituée d'un alliage de magnésium selon les revendications 1 à 5.

7. Endoprothèse selon l'une des revendications 1 à 6, **caractérisée en ce que** la structure porteuse est extrudée.

8. Endoprothèse selon la revendication 1, **caractérisée en ce que** l'endoprothèse est formée comme endoprothèse intraluminale.

9. Endoprothèse selon la revendication 8, **caractérisée en ce que** l'endoprothèse est formée comme stent.

10. Endoprothèse selon la revendication 9, **caractérisée en ce que** l'endoprothése est formée comme stent coronaire ou comme stent périphérique.

11. Endoprothèse selon la revendication 9 ou 10, **caractérisée en ce que** l'endoprothèse est formée comme stent auto-expansible ou comme stent expansible par ballon.

12. Endoprothèse selon la revendication 1, **caractérisée en ce que** la structure porteuse est fabriquée par découpe d'un tube d'une seule pièce.

13. Endoprothèse selon la revendication 1, **caractérisée en ce que** la structure porteuse est formée à partir d'un fil métallique contenant l'alliage de magnésium.

14. Endoprothèse selon la revendication 1, **caractérisée en ce que** la structure porteuse (10) entoure une cavité étendue en longueur, ouverte sur ses bouts.

15. Endoprothèse selon la revendication 14, **caractérisée en ce que** la structure porteuse (10) est en forme de treillis et formée par des brides (12, 16) ainsi que par des ouvertures radiales entourées par des brides.

16. Endoprothèse selon la revendication 15, **caractérisée en ce que** les brides (12) de la structure porteuse présentent toutes une surface de section similaire, telle que le rapport entre la plus grande et la plus petite surface de section est inférieur à 2.

17. Endoprothèse selon la revendication 15, **caractérisée en ce que** les brides (12) de la structure porteuse présentent toutes un diamètre minimal similaire, tel que le rapport entre le plus grand et le plus petit diamètre minimal est inférieur à 2.

18. Endoprothèse selon l'une des revendications 15 à 17, avec une structure porteuse (10) comprenant des anneaux de brides (14) formés par des brides (12) et des brides de raccord (16) reliant des anneaux de brides contigus, **caractérisée en ce que** les brides de raccord (16) ont une surface de section ou un diamètre minimal inférieurs aux brides (12) constituant les anneaux de brides (14).

19. Endoprothése selon la revendication 1, **caractérisée en ce que** l'endoprothése porte une substance active physiologiquement efficace.

20. Endoprothèse selon la revendication 19, **caractérisée en ce que** l'endoprothèse est revêtue d'un médicament au moins.
